(19) Europäisches Patentamt
European Patent Office
Office européen des brevets

(11) Veröffentlichungsnummer : **0 418 662 B1**

# (12) EUROPÄISCHE PATENTSCHRIFT

(45) Veröffentlichungstag der Patentschrift :
**19.05.93 Patentblatt 93/20**

(51) Int. Cl.$^5$ : **C07C 303/22,** C07C 309/46

(21) Anmeldenummer : **90117146.2**

(22) Anmeldetag : **06.09.90**

## (54) Verfahren zur Herstellung von 2-Alkylamino-4-aminobenzolsulfonsäuren.

(30) Priorität : **20.09.89 DE 3931326**

(43) Veröffentlichungstag der Anmeldung :
**27.03.91 Patentblatt 91/13**

(45) Bekanntmachung des Hinweises auf die Patenterteilung :
**19.05.93 Patentblatt 93/20**

(84) Benannte Vertragsstaaten :
**CH DE FR GB IT LI**

(56) Entgegenhaltungen :
**EP-A- 0 315 045**
**US-A- 2 575 027**
**US-A- 2 687 431**

(73) Patentinhaber : **BASF Aktiengesellschaft**
**Carl-Bosch-Strasse 38**
**W-6700 Ludwigshafen (DE)**

(72) Erfinder : **Patsch, Manfred, Dr.**
**Fritz-Wendel-Strasse 4**
**W-6706 Wachenheim (DE)**
Erfinder : **Pandl, Klaus, Dr.**
**Schumannstrasse 18**
**W-6700 Ludwigshafen (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 2-Alkylamino-4-aminobenzolsulfonsäuren durch Acylierung von 2,4-Diaminobenzolsulfonsäure, anschließende Alkylierung der resultierenden 4-Acylamino-2-aminobenzolsulfonsäure und schließlich hydrolytische Abspaltung des Acylrests.

In den älteren deutschen Patentanmeldungen P 39 14 650.2 sowie P 39 27 068.8 (später als EP-A-396 077 bzw. EP-A-413230 offenbart) sind bereits Verfahren zur Herstellung von N-alkylierten Phenylendiaminsulfonsäuren beschrieben.

Aufgabe der vorliegenden Erfindung war es, ein neues Verfahren zur Herstellung von monoalkylierten Phenylendiaminsulfonsäuren bereitzustellen, das die Zielprodukte in einfacher Weise und in guter Ausbeute liefern sollte.

Es wurde nun gefunden, daß die Herstellung von Phenylendiaminsulfonsäuren der Formel I

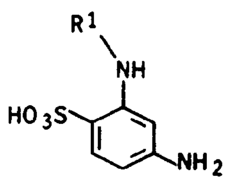 (I),

in der $R^1$ $C_1$-$C_4$-Alkyl bedeutet vorteilhaft gelingt, wenn man 2,4-Diaminobenzolsulfonsäure in einem ersten Schritt mit einem Acylierungsmittel behandelt, das sich von einer $C_2$-$C_8$-Alkansäure ableitet, und dann in einem zweiten Schritt die resultierenden Acylderivate der Formel II

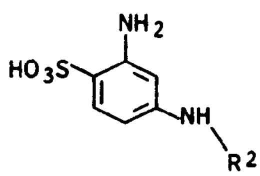 (II),

in der $R^2$ $C_2$-$C_8$-Alkanoyl bedeutet, gegebenenfalls in Gegenwart eines Alkalihalogenids oder Alkalisulfats mit einem Alkylierungsmittel der Formel III

$$R^1 - X \quad (III),$$

in der $R^1$ die obengenannte Bedeutung besitzt und X eine Abgangsgruppe bedeutet, zu Phenylendiaminderivaten der Formel IV

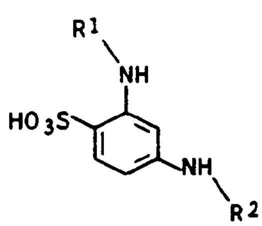 (IV),

in der $R^1$ und $R^2$ jeweils die obengenannte Bedeutung besitzen, umsetzt, und in einem dritten Schritt den $C_2$-$C_8$-Alkanoylrest hydrolytisch abspaltet.

Es ist bekannt (Organikum, 17. Auflage, Seite 201), daß Alkylierungsreaktionen an basischen Stickstoffatomen sehr unselektiv verlaufen. So entstehen bei der Umsetzung einer primären Aminoverbindung mit einem Alkylierungsmittel, z.B. mit einem Alkylhalogenid oder einem Dialkylsulfat, neben dem sekundären Amin auch in größeren Mengen tertiäre oder auch quartäre Aminoverbindungen noch bevor das Edukt vollständig abreagiert hat.

Zur Herstellung sekundärer Amine in reiner Form muß man daher häufig umständliche Reaktionsschritte wählen, z.B. Einführung geeigneter Schutzgruppen am zu alkylierenden Stickstoffatom (s. z.B. Organikum,

loc. Cit.) und hydrolytische Abspaltung der Schutzgruppe nach erfolgter Alkylierung oder Abscheiden des sekundären Amins als schwerlösliches Nitrosamin aus der Reaktionslösung und anschließende hydrolytische Aufarbeitung des isolierten Nitrosamins (s. z.B. Houben-Weyl, Methoden der Organischen Chemie, Band 11/1, Seiten 207 bis 212).

Es war nun überraschend, daß beim erfindungsgemäßen Verfahren eine gezielte Monoalkylierung der $NH_2$-Gruppe stattfindet.

Das erfindungsgemäße Verfahren wird im allgemeinen so durchgeführt, daß man zunächst 2,4-Diaminobenzolsulfonsäure mit dem Acylierungsmittel in wäßrigem Reaktionsmedium umsetzt.

Bei den genannten Acylierungsmitteln, die sich von einer $C_2$-$C_8$-Alkansäure ableiten, handelt es sich üblicherweise um die entsprechenden Carbonsäureanhydride, wobei auch gemischte Carbonsäureanhydride in Betracht kommen, oder um die entsprechenden Carbonsäurehalogenide. Beispielhaft seien Essigsäureanhydrid, Propionsäureanhydrid, Acetylchlorid oder Propionylchlorid genannt. Die Verwendung von Propionsäureanhydrid oder Essigsäureanhydrid, insbesondere aber von Essigsäureanhydrid, ist bevorzugt.

Je Mol 2,4-Diaminobenzolsulfonsäure werden üblicherweise 1,0 bis 1,5 Mol, vorzugsweise 1,05 bis 1,15 Mol des Acylierungsmittels verwendet. Im allgemeinen läuft die Acylierung bei einer Temperatur von 10 bis 45°C, vorzugsweise 20 bis 40°C und bei einem pH-Wert von 3 bis 7,5 ab.

Nach beendeter Umsetzung, die im allgemeinen 1 bis 6 Stunden in Anspruch nimmt, kann das Acylierungsprodukt der Formel II bei einem pH-Wert von 0,5 bis 7,5 z.B. mittels Kochsalz ausgefällt werden. Es ist jedoch auch möglich, die anfallende Reaktionslosung direkt weiterzuverarbeiten.

Die Phenylendiaminderivate der Formel IV erhält man durch Umsetzung des Acylierungsproduktes II mit einem Alkylierungsmittel der Formel III

$$R^1 - X \quad \text{(III)},$$

in der $R^1$ $C_1$-$C_4$-Alkyl und X eine Abgangsgruppe bedeuten, gegebenenfalls in Gegenwart eines Alkalihalogenids oder Alkalisulfats, wobei in diesem Fall die Verwendung von Alkalichlorid oder Alkalisulfat hervorzuheben ist.

Geeignete Abgangsgruppen X sind z.B. Halogen, wie Chlor, Brom oder Iod, oder ein Rest der Formel $O$-$SO_2$-$C_6H_5$, $O$-$SO_2$-$C_6H_4$-$CH_3$ oder $O$-$SO_2$-$OR^3$, wobei $R^3$ für $C_1$-$C_4$-Alkyl steht. Die Verwendung von $C_1$-$C_4$-Dialkylsulfaten, insbesondere von Dimethylsulfat ist bevorzugt.

Die Alkylierung wird nach an sich bekannten Methoden in wäßrigem Reaktionsmedium durchgeführt. Je Mol Acylierungsprodukt II verwendet man im allgemeinen 1,0 bis 3,0 Mol, vorzugsweise 1,1 bis 2,1 Mol des Alkylierungsmittels III. Der pH-Wert der Reaktion beträgt üblicherweise 2,0 bis 10,0, vorzugsweise 3,5 bis 7,0, und kann gegebenenfalls durch Zugabe einer Base, z.B. Natrium- oder Kaliumhydroxid, Natrium- oder Kaliumcarbonat oder -hydrogencarbonat oder Natriumacetat, im gewünschten Bereich gehalten werden.

Die Reaktionstemperatur beträgt üblicherweise 10 bis 50°C. Beim Arbeiten mit Dimethylsulfat ist eine Temperatur von 10 bis 30°C bevorzugt.

Eine bevorzugte Verfahrensweise besteht darin, daß man im zweiten Schritt die Alkylierung des Acylderivats der Formel II bei einer Anfangskonzentration von 0,5 bis 1,7 Mol Acylderivat der Formel II, bezogen auf 1 Liter Reaktionsgemisch, vornimmt.

Besonders bevorzugt ist das Verfahren, wenn man vor Beginn der Alkylierungsreaktion dem Reaktionsgemisch 50 bis 250 g, jeweils bezogen auf 1 Mol Acylderivat II, eines Alkalichlorids oder Alkalisulfats zusetzt. Geeignete Salze sind z.B. Lithiumchlorid, Natriumchlorid, Kaliumchlorid, Natriumsulfat, Kaliumsulfat, Natriumhydrogensulfat oder Kaliumhydrogensulfat, wobei der Verwendung von Natrium- oder Kaliumchlorid oder Natriumsulfat besondere Bedeutung zukommt.

Die Umsetzung erfolgt zweckmäßig durch Zugabe des Alkylierungsmittels zur Reaktionslösung. Die Zugabezeit beträgt im allgemeinen 30 Minuten bis 5 Stunden.

Das Reaktionsprodukt IV kann durch einfaches Abfiltrieren, gegebenenfalls nach Aussalzen, bei einem pH-Wert von 0 bis 7 isoliert werden. Man kann aber auch ohne Zwischenisolierung die $C_2$-$C_8$-Alkanoylgruppe hydrolytisch abspalten. Dies kann entweder im sauren oder alkalisch wäßrigen Milieu geschehen, wobei die saure Hydrolyse bevorzugt ist. Geeignete Medien sind z.B. Salzsäure, eine wäßrige Schwefelsäurelösung, Natronlauge oder Kalilauge.

Sowohl im sauren als auch im alkalischen Milieu wird die hydrolytische Abspaltung unter Erwärmen auf Rückflußtemperatur vorgenommen. Nach einer Reaktionsdauer von 2 bis 3 Stunden ist die Abspaltung zu Ende und man kann das Zielprodukt nach an sich bekannten Methoden isolieren.

Mittels des neuen Verfahrens, das sowohl in kontinuierlicher als auch in diskontinuierlicher Arbeitsweise vorgenommen werden kann, gelangt man in einfacher Weise zu den Phenylendiaminsulfonsäuren der Formel I. Bei ihnen handelt es sich um wertvolle Zwischenprodukte für die Synthese von Farbstoffen, z.B. von Reaktivfarbstoffen, wie sie in der EP-A-315 045 oder EP-A-315 046 beschrieben sind.

Die folgenden Beispiele sollen die Erfindung näher erläutern.

Beispiel

235 g 2,4-Diaminobenzolsulfonsäure wurden in 1000 ml Wasser durch Zugabe von Natronlauge bei einem pH-Wert von 7 gelöst. Zur Lösung wurden 84 g Natriumhydrogencarbonat und 53 g Natriumcarbonat und innerhalb von 5 Stunden bei 25°C 162 g Essigsäureanhydrid gegeben. Nach 1 Stunde Nachrühren wurden 300 g Natriumchlorid hinzugegeben und bei 30°C bis 35°C 332 g Dimethylsulfat zudosiert. Danach wurde 30 Minuten nachgerührt und mit Salzsäure ein pH-Wert von 0 bis 0,5 eingestellt. Anschließend wurde 3 Stunden unter Rückfluß erhitzt, unter Rühren abgekühlt, mit Natronlauge ein pH-Wert von 1,0 eingestellt und filtriert.

Ausbeute: 202 g 2-Methylamino-4-aminobenzolsulfonsäure.

Beispiel 2

1,88 kg 2,4-Diaminobenzolsulfonsäure wurden in 2,5 l Wasser durch Zugabe von Natronlauge bei einem pH-Wert von 7 gelöst. Innerhalb von 3 Stunden wurden bei 20°C 1,08 kg Essigsäureanhydrid hinzugetropft. Mit Natronlauge wurde der pH-Wert auf 7 gestellt und dann 0, 17 kg Natriumhydrogencarbonat sowie 0,9 kg Natriumsulfat hinzugegeben. Bei 25°C wurden dann 1,6 kg Dimethylsulfat zudosiert. Anschließend wurde 30 Minuten nachgerührt und mit Salzsäure ein pH-Wert von 0 eingestellt. Danach wurde mit 3,5 l Wasser verdünnt, 3 Stunden unter Rückfluß erhitzt, unter Rühren abgekühlt, mit Natronlauge ein pH-Wert von 0,5 eingestellt und filtriert.

Ausbeute: 1,55 kg 2-Methylamino-4-aminobenzolsulfonsäure.

**Patentansprüche**

1. Verfahren zur Herstellung von Phenylendiaminsulfonsäuren der Formel I

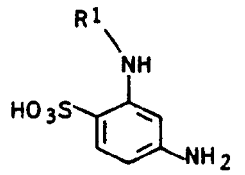

(I),

in der $R^1$ $C_1$-$C_4$-Alkyl bedeutet, dadurch gekennzeichnet, daß man 2,4-Diaminobenzolsulfonsäure in einem ersten Schritt mit einem Acylierungsmittel behandelt, das sich von einer $C_2$-$C_8$-Alkansäure ableitet, und dann in einem zweiten Schritt die resultierenden Acylderivate der Formel II

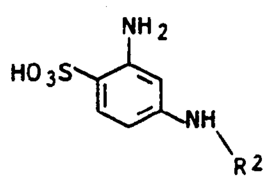

(II),

in der $R^2$ $C_2$-$C_8$-Alkanoyl bedeutet, gegebenenfalls in Gegenwart eines Alkalihalogenids oder Alkalisulfats mit einem Alkylierungsmittel der Formel III

$$R^1 - X \quad \text{(III)},$$

in der $R^1$ die obengenannte Bedeutung besitzt und X eine Abgangsgruppe bedeutet, zu Phenylendiaminderivaten der Formel IV

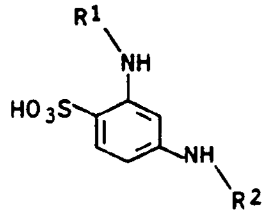

in der $R^1$ und $R^2$ jeweils die obengenannte Bedeutung besitzen, umsetzt, und in einem dritten Schritt den $C_2$-$C_8$-Alkanoylrest hydrolytisch abspaltet.

2. Verfahren nach Anspruch 1, dadurch gekennzeichnet, daß man im zweiten Schritt die Alkylierung des Acylderivats der Formel II bei einer Anfangskonzentration von 0,5 bis 1,7 Mol Acylderivat der Formel II, bezogen auf 1 Liter Reaktionsgemisch, vornimmt.

3. Verfahren nach Anspruch 1, dadurch gekennzeichnet, daß man dem Reaktionsgemisch vor Beginn der Alkylierungsreaktion 50 bis 250 g, bezogen auf 1 Mol Acylderivat der Formel II, eines Alkalichlorids oder Alkalisulfats zusetzt.

## Claims

1. A process for the preparation of a phenylenediaminesulfonic acid of the formula I

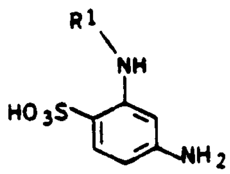

(I)

where $R^1$ is $C_1$-$C_4$-alkyl, wherein 2,4-diaminobenzenesulfonic acid is treated in a first step with an acylating agent which is derived from a $C_2$-$C_8$-alkanoic acid, and then, in a second step, the resulting acyl derivative of the formula II

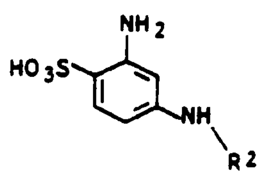

(II)

where $R^2$ is $C_2$-$C_8$-alkanoyl, is reacted, in the presence or absence of an alkali metal halide or of an alkali metal sulfate, with an alkylating agent of the formula III

$$R^1 - X \quad \text{(III)},$$

where $R^1$ has the abovementioned meanings and X is a leaving group, to give a phenylenediamine derivative of the formula IV

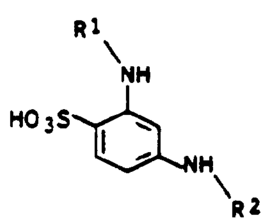

where $R^1$ and $R^2$ each have the abovementioned meaning, and, in a third step, the $C_2$-$C_8$-alkanoyl radical is eliminated by hydrolysis.

2. A process as claimed in claim 1, wherein, in the second step, the alkylation of the acyl derivative of the formula II is carried out at an initial concentration of from 0.5 to 1.7 mol, based on 1 liter of reaction mixture, of the acyl derivative of the formula II.

3. A process as claimed in claim 1, wherein from 50 to 250 g, based on 1 mol of acyl derivative of the formula II, of an alkali metal chloride or of an alkali metal sulfate are added to the reaction mixture before the beginning of the alkylation reaction.

**Revendications**

1. Procédé de préparation d'acides phénylènediaminosulfoniques de formule I

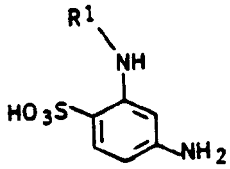

(I),

dans laquelle $R^1$ représente un reste alkyle en $C_1$-$C_4$, caractérisé en ce que, dans une première étape, on traite des acides 2,4-diaminobenzènesulfoniques par un agent d'acylation qui dérive d'un acide alcanoïque en $C_2$-$C_8$, puis, dans une deuxième étape, on fait réagir les dérivés acylés résultants de formule II

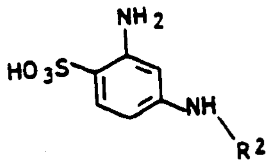

(II),

dans laquelle $R^2$ représente un reste alcanoyle en $C_2$-$C_8$, éventuellement en présence d'un halogénure alcalin ou d'un sulfate alcalin, avec un agent d'alkylation de formule III

$R^1$ - X (III)

dans laquelle $R^1$ a la signification sus-indiquée et X représente un groupement enlevable, pour obtenir des dérivés phénylènediaminés de formule IV

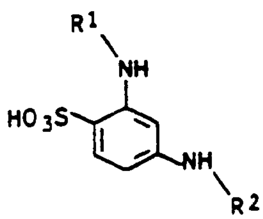

dans laquelle $R^1$ et $R^2$ ont chacun la signification sus-indiquée, et, dans une troisième étape, on enlève le reste alcanoyle en $C_2$-$C_8$ par voie d'hydrolyse.

2. Procédé selon la revendication 1, caractérisé en ce que, dans la deuxième étape, on procède à l'alkylation du dérivé acylé de formule II à une concentration initiale de 0,5 à 1,7 mole de dérivé acylé de formule II pour 1 litre de mélange réactionnel.

3. Procédé selon la revendication 1, caractérisé en ce qu'avant le début de la réaction d'alkylation, on ajoute au mélange réactionnel 50 à 250 g, par rapport à 1 mole de dérivé acylé de formule II, d'un chlorure alcalin ou d'un sulfate alcalin.